# EUROPEAN PATENT SPECIFICATION

(11) **EP 2 634 515 B1**
(45) Date of publication and mention of the grant of the patent: **04.09.2019**
(21) Application number: 12157479.2
(22) Date of filing: 29.02.2012
(51) Int. Cl.: F25D 17/04

(54) **Blast chiller apparatus and a method to sanitize a blast chiller apparatus**
Schockkühlervorrichtung und Verfahren zum Säubern einer Schockkühlervorrichtung
Appareil de refroidissement rapide et procédé d'assainissement d'un appareil de refroidissement rapide

(43) Date of publication of application: 04.09.2013
(73) Proprietor: Electrolux Professional S.p.A., 33170 Pordenone (IT)
(72) Inventor: Camatta, Massimiliano, 33170 Pordenone (IT); Pacorich, Massimo, 33170 Pordenone (IT); Reale, Fabio, 33170 Pordenone (IT); Sinatra, Fabio, 33170 Pordenone (IT)
(74) Representative: Electrolux Group Patents

(56) References cited:
- EP-A2- 0 312 060
- WO-A1-2010/070432
- DE-A1- 19 749 971
- JP-A- 8 296 938
- US-A- 5 052 191
- US-A1- 2005 178 984

## Description

The present invention relates to a blast chiller apparatus and a method to sanitize a blast chiller apparatus.

More specifically, the present invention relates to a blast chiller apparatus, which comprises a chilling chamber structured for housing food to be cooled, and is configured to quickly cool and/or freeze food arranged inside the chilling chamber, to which the following description refers purely by way of example without implying any loss of generality.

It should be pointed out that hereafter with the terms "sanitize", it will be meant those factors that improve the general cleanliness of the chilling chamber of the chiller apparatus and aid to the preservation of health. In detail, the terms "sanitize" or "sanitization" as used herein means to free a targeted object from contaminants particularly in the form of living organisms such as the microorganisms.

As it is known, a most efficient way to "sanitize" an object, i.e. eliminate moulds, yeasts, bacterial spores and/or similar, consists in subjecting these micro-organisms to the action of ultraviolet radiations, as may be generated, for example, by UV sources/lamps currently available on the market.

One of the more common types of UV lamps that are used as sanitizing agents is the low pressure mercury lamp. Low pressure mercury lamps are generally cost effective in that their power requirements are low as compared to other types of UV light sources, yet low pressure mercury lamps also have a comparatively high UV output.

In view of the above advantages, UV mercury lamps have been proven to be effective in sanitizing the chilling chamber of blast chiller apparatus.

However, degradation in the performance of the UV mercury lamps operating inside of the chilling chamber is experienced due to the colder temperatures that fall well below the optimal operating temperatures of the UV mercury lamps.

As a matter of the fact, UV mercury lamps are unable to adequately function in cooling/freezing temperatures that, as it is known, fall outside optimal operating range of temperatures of the mercury lamps.

In order to perform the sanitizing cycle maintaining reasonable performance of the UV mercury lamps, in the blast chiller apparatus above disclosed, it is needed to open the door of the chilling chamber to cause the temperature inside of the chamber to grow up during a rest period of the chiller, so that the temperature inside of the chilling chamber reaches the outside temperature corresponding to the environment temperature.

When temperature inside the chilling chamber has reached the environment temperature, i.e. 15° C, the sanitizing cycle by UV lamp starts. Temperature reached inside of the chilling chamber in such conditions slightly improves the operating performance of UV mercury lamps.

However, environment temperature is usually lower than optimal operating range of temperatures, thus UV mercury lamps work with low efficiency, causing a partial degradation in the sanitizing performance.

Moreover, the sanitization operations need long time to be performed because, after opening the chiller-door, it is necessary to wait that temperature inside of cooling chamber reaches the environment temperature.

In addition, blast chiller apparatus above disclosed are typically provided with a chilling circuit comprising an evaporator which is arranged inside the chilling chamber and is structured to rapidly cool/freeze the latter.

However, the evaporator presents parts of its cooling surface which are shielded by means of an outer cover and thus are not irradiated from ultraviolet radiations, causing an incomplete sanitization of the microorganism that, as a consequence, remain on the evaporator shielded surfaces, affecting food preservability.

US 2005 0178 984 discloses a heat controlled ultraviolet light apparatus including a source of ultraviolet light, a cover, and a heating or cooling element that heats/cools the space between the ultraviolet light source and the cover.

Although ultraviolet light apparatus disclosed in US 2005 0178 984 is structured to optimize the output of the ultraviolet light source regardless of temperature at which the source is exposed during use, it is expensive and complex and is not able to irradiate microorganisms present on the evaporator shielded surfaces.

US 6 237 250 discloses a refrigerated display case, including a goods platform, a goods space arranged above the platform, a tub space that is arranged below the platform and is bordered by a display case tub, a blower, an evaporating apparatus and a UV sterilizing tube. The evaporating apparatus is arranged in the effective range of the UV sterilizing tube, which admits the evaporating apparatus during the defrosting, carried out with the aid of a hot-gas defrosting device. During the relatively short defrosting phase, the temperature of the goods changes only insignificantly, that is to say by only 0.5 to 1° C.

Refrigerated display case disclosed in US 6 237 250 is not able to sanitize completely all the evaporator surfaces.

In-depth research has been carried out by the applicant to achieve the following specific goals:
- reduce sanitization time;
- guarantee high performance of the UV light sources, in particular UV mercury lamps;
- perform sanitization of the closed chilling chamber during an operating phase usually performed by the blast chiller apparatus, so that the system is simplified;
- sanitize completely all the evaporator surfaces.

It is therefore an object of the present invention to provide a solution designed to achieve the above goals.

JP 8 296 938 discloses a blast chiller apparatus comprising a chilling chamber structured for housing food to be cooled/frozen, a chilling circuit comprising a heat exchanger designed to rapidly cool/freeze the chilling chamber.

EP 0 312 060 discloses an automatic refrigeration apparatus including a storage compartment intended for keeping foodstuffs, or other items, at a required temperature situated anywhere between approx. 0 DEG C and 10 DEG C. The apparatus comprising a germicide means 12 which can consist of at least one UV lamp capable of generating C- type ultraviolet radiations, i.e. radiations having a wavelength equal to 2537 A/ DEG .

WO 2010 070 432 discloses a device and a method for rapidly sterilizing liquid nitrogen in a container using ultraviolet radiation irradiated for a predetermined length of time based on the temperature measured by a sensor and on the minimum dose of radiation necessary to kill micro-organisms resistant to liquid nitrogen

US 5 052 191 discloses a method and apparatus for defrosting the outside heat exchanger of a reversible vapor compression refrigeration (heat pump) system, wherein a heat storage apparatus is mounted in heat exchange relationship with the refrigerant piping between the system flow reversing valve and the inside heat exchanger.

The present invention is disclosed in the independent claims 1 and 10. Further embodiments are disclosed in the dependent claims.

Preferably the prefixed optimum temperature range is between about 30°C and 50°C.

Preferably, the prefixed sanitizing temperature range is between about 50°C and 80°C, more preferably between about 50°C and 80°C.

Preferably the chilling circuit comprises a reversible-cycle heat pump assembly, which is provided with said first heat exchanger and is designed to work to perform a direct thermal cycle to rapidly cool/freeze the chilling chamber or, alternately, a reversed thermal cycle, to cause said first heat exchanger to be heated; during said reversed thermal cycle, said reversible-cycle heat pump assembly being said defrost system.

Preferably, the chilling circuit is provided with a heat pump assembly comprising refrigerant compressing means; said defrost system comprising a bypass channel which is interposed between the refrigerant-outlet of the refrigerant compressing means and the refrigerant-inlet of said first heat exchanger; and valve means arranged along said bypass channel for connecting, based on a command, the refrigerant-outlet of the refrigerant compressing means to the refrigerant-inlet of the first heat exchanger; said control system being configured to control said valve means to defrost said first heat exchanger.

Preferably, the defrost system comprise electric heating means associated with said first heat exchanger; said control system being configured to control said electric heating means to defrost said first heat exchanger.

A non-limiting embodiment of the present invention will be described by way of example with reference to the accompanying drawings, in which:
Figure 1 shows a schematic lateral cross section of a blast chiller apparatus, according to the present invention;
Figure 2 shows a schematic lateral cross section of a blast chiller apparatus, according to a first different embodiment of the present invention;
Figure 3 shows a schematic lateral cross section of a blast chiller apparatus, according to a second different embodiment of the present invention.

With reference to accompanying Figure, referral number 1 indicates as a whole a blast refrigerating/freezing apparatus such as a professional or domestic/household blast chiller apparatus, which is structured to store food and is configured to quickly cooling or rapidly freezing the stored food.

It should be pointed out that hereafter with the terms "blast chiller apparatus", it will be meant a professional or household blast chiller configured to bring the temperature of food, such as for example cooked food, from about +90°C/60°C to about + 3°C in a short time, or a professional or household blast freezing chiller configured to bring the temperature of the food from about + 90°C/ 60°C to about - 18 °C in a short time.

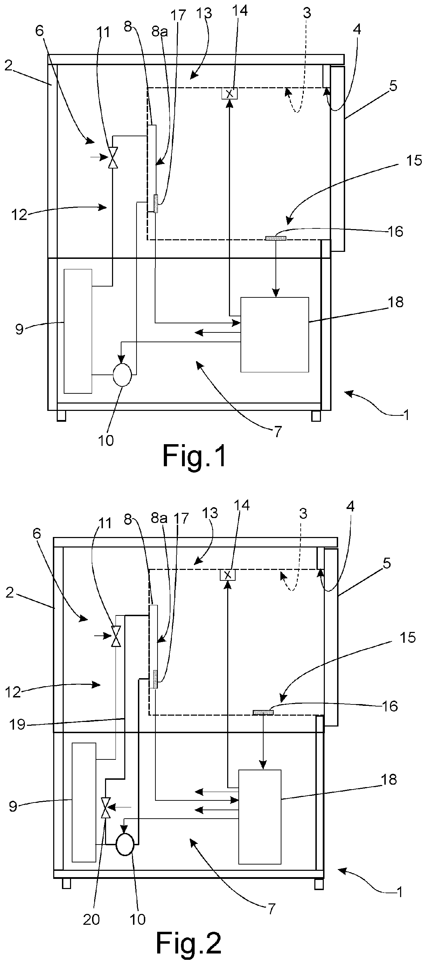

Referring to Figure 1, blast chiller 1 comprises a preferably, though not necessarily, parallelepiped-shaped outer boxlike casing 2 structured for resting on the floor; a chilling chamber 3 which is structured for internally housing the food to be cooled, and which is located inside the outer casing 2, directly facing an access opening 4 preferably, though not necessarily, realized in the front wall of casing 2; and a porthole door 5 hinged to the front wall of casing 2 to rotate about a preferably, though not necessarily, vertically-oriented reference axis, to and from a closing position in which door 4 rests completely against the front wall to close the access opening 4.

According to a preferred embodiment of the present invention, the blast chiller apparatus 1 is also provided with a heat-pump type, chilling circuit 6 which is located inside the outer casing 2 and is structured to quickly cooling or rapidly freezing the chilling chamber 3.

Preferably, the heat-pump type, chilling circuit 6 is provided with a heat-pump assembly 7 comprising:
- a first heat exchanger 8, which is located at least partially inside of the chilling chamber 3 and is designed so that the refrigerant fluid absorbs heat from air presents inside of the chilling chamber 3 in order to rapidly cooling down the food placed in the chamber 3;
- a second heat exchanger 9, which is located outside of the chilling chamber 3 along the circulation refrigerant circuit of the heat-pump assembly 7, and which is designed to release heat absorbed by the refrigerant into the chilling chamber 3;
- an electrically-powered refrigerant compressing device 10, which is interposed between the refrigerant-outlet of the first heat exchanger 8 and the refrigerant-inlet of the second heat exchanger 9, and which is structured for compressing the gaseous-state refrigerant directed towards the heat exchanger 9 so that refrigerant pressure and temperature are much higher at the refrigerant-inlet of second heat exchanger 9 than at the refrigerant-outlet of the first heat exchanger 8; and
- an expansion valve 11 or similar passive/operated refrigerant expansion device (for example a capillary tube, a thermostatic valve or an electrically-controlled expansion valve) which is interposed between the refrigerant-outlet of the second heat exchanger 9 and the refrigerant-inlet of the first heat exchanger 8, and is structured so as to cause a rapid expansion of the refrigerant directed towards the first heat exchanger 8, so that refrigerant pressure and temperature are much higher at the refrigerant-outlet of the second heat exchanger 9 than at the refrigerant-inlet of the first heat exchanger 8.

The first heat exchanger 8, conventionally referred to as "evaporator" is structured so that the low-pressure and low-temperature refrigerant directed to the refrigerant compressing device 10 absorbs heat from the air present inside of the chilling chamber 3, thus causing cooling of the latter, while the second heat exchanger 9, conventionally referred to as "condenser", is structured so that the high-temperature refrigerant arriving from the delivery of the refrigerant compressing device 10 releases heat to the environment. The refrigerant compressing device 10, whose function is clear from the above, is conventionally referred to as "compressor".

According to a preferred embodiment of the present invention the heat-pump assembly 7 may be a "reversible-cycle heat pump" assembly designed to work either in a first operating state based on a direct thermal cycle, wherein the first heat exchanger 8 works as an "evaporator" in order to cool the chilling chamber 3 and the second heat exchanger 9 works as a "condenser" to release the absorbed heat, or in a second operating state based on a reversed thermal cycle, wherein the first heat exchanger 8 works as a "condenser" in order to release heat of the refrigerant to the cooling surfaces 8a of the first heat exchanger 8 to defrost the latter, and the second heat exchanger 9 operates as an "evaporator".

According to Figure 1, the blast chiller apparatus further comprises a sanitizing system 13 configured to sanitize the chilling chamber 3 and/or the evaporator's surfaces 8a faced to the inner space of the chilling chamber 3.

The sanitizing system 13 comprises a defrost system 12, which is designed to defrost the first heat exchanger 8, and at least a UV-source 14, which is preferably, though not necessarily, arranged inside of the chilling chamber 3 and is designed to generate ultraviolet radiation towards the inner surfaces of the chilling chamber 3 to be sanitized so that microorganisms are destroyed and/or inactivated.

Referring to the embodiment shown in Figure 1, the defrost system 12 is defined by the heat-pump assembly 7 working in the second operating state, i.e. in the reversed thermal cycle, and is designed to defrost the cooling surfaces 8a of the first heat exchanger 8 faced to the inner space of the chilling chamber 3.

The UV-source 14 comprises preferably a UV-C mercury lamp designed to generate C-type ultraviolet radiations.

According to the present invention, the sanitizing system 13 further comprises an electronic control system 15 which is configured to control the defrost system 12 to heat the first heat exchanger 8 in order to have the temperature inside of the chilling chamber 3 within a prefixed optimum temperature range of operating of the UV source 14.

The electronic control system 15 is preferably further configured to control the defrost system 12 so that the temperature of the first heat exchanger 8 is within a prefixed sanitizing temperature range in order to cause a thermal sanitization of the first heat exchanger 8.

In detail, the electronic control system 15 is configured to control the defrost system 12 to defrost the first heat exchanger 8 and cause, during the defrost phase, the inner temperature of the chamber 3 to grow up until it reaches a first prefixed temperature associated with the prefixed optimum range of operating of the UV-source 14. The electronic control system 15 is preferably configured also to cause, during the defrost phase, the temperature of the first heat exchanger 8, in particular of its surfaces 8a, to grow up until it reaches a second prefixed temperature comprised in the prefixed sanitizing temperature. Moreover, the electronic control system 15 is configured to switch-on the UV source 14 to generate ultraviolet radiations, when said first and/or second prefixed temperatures are reached.

Preferably, the electronic control system 15 comprises a temperature sensing/measuring device 16 designed to measure the temperature inside of the chilling chamber 3, and/or a temperature sensing/measuring device 17 designed to measure the temperature of the surfaces 8a of the first heat exchanger 8. Moreover, the electronic control system 15 preferably comprises also an electronic control unit 18 which is configured to: receive in input the temperatures measured by the temperature measuring devices 16 and 17; operate the heat-pump assembly 7 to cause transition of the latter between the first and the second operating states, in particular from the first to the second operating state when the first heat exchanger 8 is to be defrosted; control the heat-pump assembly 7 during defrost phase based on the measured temperatures, so that the inner temperature of the chilling chamber 3 is raised up to the first prefixed temperature, and the temperature of the cooling surfaces 8a of the first heat exchanger 8 is raised up to the second prefixed temperature; and finally switch-on the UV source 14 when said first and/or second prefixed temperatures are reached.

The first prefixed temperature falls in the prefixed optimum temperature range of the UV-source 14, which is preferably between about 30°C and 50° C. A suitable first prefixed temperature is for example about 40°C.

The second prefixed temperature associated with the prefixed sanitizing temperature range is preferably between about 50°C and 80°C, more preferably between about 60°C and 80° C. A suitable second prefixed temperature is for example about 70°C.

According to a different embodiment of the present invention shown in Figure 2, the heat-pump assembly 7 comprises a bypass channel 19 connecting the refrigerant-outlet of the refrigerant compressing device 10 with the refrigerant-inlet of the first heat exchanger 8, and an electric valve 20, in particular a two-way valve, arranged along the bypass channel 19, and designed to operate, on command, between a closed state wherein it closes the bypass channel 19 to cause the heat-pump assembly 7 to operate in the first operating state above disclosed, and an open state, wherein the electric valve 20 connects the refrigerant-outlet of the compressing device 10 to the refrigerant-inlet of the first heat exchanger 8 so that the latter receives heated refrigerant compressed by the compressing device 10 and is subjected to defrost.

More specifically, the heated refrigerant causes defrost of the surfaces 8a of the first heat exchanger 8. As a consequences, the first heat exchanger 8, the refrigerant compressing device 10, the refrigerant channel connecting the refrigerant output of the first heat exchanger 8 to the refrigerant-input of the compressing device 10, the bypass channel 19 and the electric valve 20 define the defrost system 12.

According to the embodiment shown in Figure 2, the electronic control unit 18 is configured to supply control signals to the electric valve 20 and to the expansion valve 11. When the first heat exchanger 8 is to be defrosted, the electronic control unit 18 sends control signals to close the expansion valve 11 and to open the electric valve 20. By opening the electric valve 20, the refrigerant-outlet of the compressing device 10 is connected to the refrigerant-inlet of the first heat exchanger 8 so that defrost of the latter, namely defrost of the evaporator, is started. The electronic control unit 18 is further configured to control the refrigerant compressing device 10 in order to cause, during defrost phase, raising of the inner temperature of the chamber 3 up to the first prefixed temperature and preferably also raising of the temperature of surfaces 8a up to the second prefixed temperature.

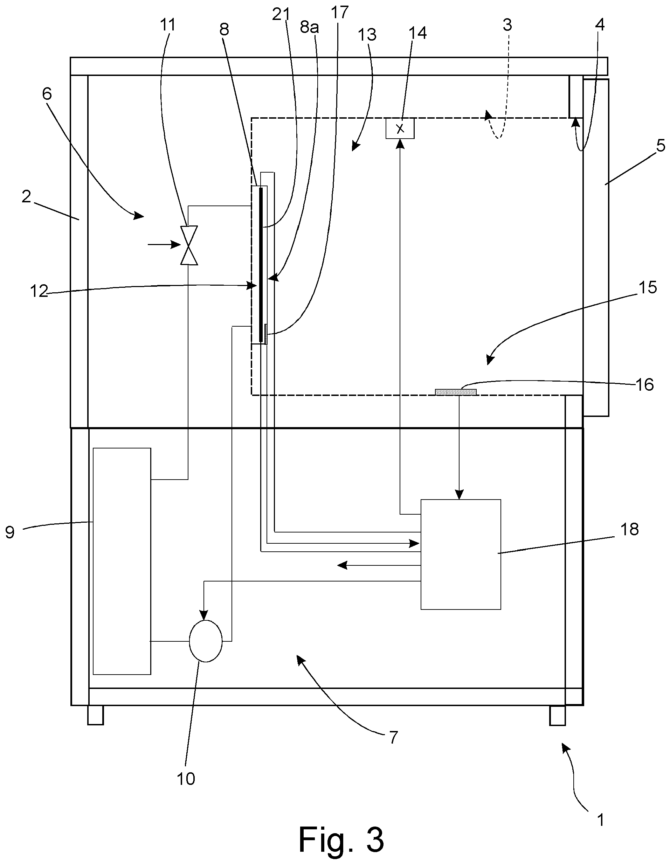

According to the different embodiment of the present invention shown in Figure 3, the defrost system 12 comprises heating means 21 configured to heat the evaporator surfaces 8a in order to quickly defrost the latter.

Preferably, the heating means 21 may comprise at least an electric component, i.e. a resistor or similar, integrated with, coupled to, or arranged close to, the first heat exchanger 8 to cause defrost of the latter.

However, it should to be noted that heating means 21 may comprise any known system which is able to release heat to the evaporator surfaces 8a in order to cause defrosting of the latter.

In the embodiment of Figure 3, the electronic control unit 18 is configured to control the operations of the heat-pump assembly 7, of the heating means 21 and of the UV source 14.

In particular, in order to sanitize the chilling chamber 3, the electronic control unit 18 is configured to switch off the heat-pump assembly 7; switch-on the heating means 21 to start defrosting, and to control, during the defrost phase, the heating means 21 (for example by performing a current-control, or voltage control, or power-control) based on the measured temperature(s), to raise the inner temperature of the chilling chamber 3 up to the first prefixed temperature and, preferably, also the surface temperature of evaporator surfaces 8a up to the second prefixed temperature. The electronic control unit 18 is also configured to switch-on the UV source 14 to generate ultraviolet radiations, when the first and/or second prefixed temperatures are reached.

Possibly, the defrosting technique according to Figure 3, making use of heating means 21, can be used in combination with one of the techniques according to Figures 1 and 2.

The blast chiller apparatus according to the present invention has the major advantages of:
- reducing sanitization time; as a matter of the fact, sanitization is made during defrost phase;
- guarantying high performance of the UV light sources, because the latter works in the optimal operating range of temperatures of the mercury lamps;
- being simple to be performed;
- sanitizing completely all the evaporator surfaces; as a matter of the fact evaporator surfaces are subjected to a thermal sanitization treatment.

## Claims

1. A blast chiller apparatus (1) comprising a chilling chamber (3) structured for housing food to be cooled/frozen, a chilling circuit (6) comprising at least a first heat exchanger (8) designed to cool/freeze the chilling chamber (3); **characterized in** comprising a sanitizing system (13) which is configured to sanitize said chilling chamber (3) by destroying and/or inactivating microorganisms contaminating said chilling chamber (3), said sanitizing system (13) comprising:
- a defrost system (12) designed to defrost said first heat exchanger (8);
- at least an UV source (14) designed to irradiate with ultraviolet radiations the inner surfaces of said chilling chamber (3); and
- a control system (15) configured to control said defrost system (12) to heat the first heat exchanger (8) so as to cause the inner temperature of the chilling chamber (3) to grow up until it is comprised within a prefixed first temperature range associated with a high performance operating condition of said UV source (14) and being further configured to switch on said UV source (14) when the inner temperature of the chilling chamber (3) falls in said prefixed first temperature range.

2. Blast chiller apparatus according to Claim 1, wherein said control system (15) is configured to control the defrost system (12) to heat the first heat exchanger (8) so as to have or maintain simultaneously the temperature of said first heat exchanger (8) in a prefixed second temperature range associated with a thermal sanitization condition of said heat exchanger (8) and the temperature of the chilling chamber (3) in said prefixed first temperature range, and is further configured to switch on said UV source (14) when the temperature of the first heat exchanger (8) falls in said prefixed second temperature range and the inner temperature of the chilling chamber (3) falls in said prefixed first temperature range.

3. Blast chiller apparatus according to Claim 1 or 2, wherein the control system (15) comprises at least a temperature sensor (16,17) configured to measure the temperature inside of said chilling chamber (3); the control system (15) being further configured to switch-on said UV source (14) to generate ultraviolet radiations when said measured temperature falls in said prefixed first temperature range.

4. Blast chiller apparatus according to any of the preceding Claims, wherein said prefixed first temperature range is between about 30°C and 50°C.

5. Blast chiller apparatus according to Claim 2, wherein said prefixed second temperature range is between about 50°C and 80°C.

6. Blast chiller apparatus according to Claim 5, wherein said prefixed second temperature range is between about 60°C and 80°C.

7. Blast chiller apparatus according to any of the preceding Claims, wherein said chilling circuit (6) comprises a reversible-cycle heat pump assembly (7), which comprises said first heat exchanger (8) and is designed to work to perform a direct thermal cycle to cool said first heat exchanger (8) so as to rapidly cool/freeze the chilling chamber (3) or, alternately, a reversed thermal cycle, to heat said first heat exchanger (8), so as to defrost said first heat exchanger (8).

8. Blast chiller apparatus according to any of the Claims from 1 to 6, wherein said chilling circuit (6) is provided with a heat pump assembly (7) comprising refrigerant compressing means (10); a bypass channel (19) which is interposed between the refrigerant-outlet of the refrigerant compressing means (10) and the refrigerant-inlet of said first heat exchanger (8); and valve means (20) arranged along said bypass channel (19) for connecting, based on a command, the refrigerant-outlet of the refrigerant compressing means (10) to the refrigerant-inlet of the first heat exchanger (8); said control system (15) being configured to control said valve means (20) to defrost said first heat exchanger (8).

9. Blast chiller apparatus according to any of the Claims from 1 to 8, wherein said defrost system (12) comprises electric heating means (21) associated with said first heat exchanger (8); said control system being configured to control said electric heating means (21) to defrost said first heat exchanger (8).

10. Method to sanitize a blast chiller apparatus (1) comprising a chilling chamber (3) structured for housing food to be cooled/frozen, a chilling circuit (6) comprising at least a first heat exchanger (8) designed to rapidly cool/freeze the chilling chamber (3); **characterized in that** there is a sanitizing system (13) comprising a defrost system (12) designed to defrost said at least first heat exchanger (8); and at least an UV source (14) designed to irradiate with ultraviolet radiations the inner surfaces of said chilling chamber (3); the method comprising the step of sanitizing said chilling chamber (3) by destroying and/or inactivating microorganisms contaminating said chilling chamber (3) by the sanitizing system (13), said step of sanitizing comprising the step of controlling the defrost system (12) to heat the first heat exchanger (8) so as to cause the inner temperature of the chilling chamber (3) to grow up until it is comprised within a prefixed first temperature range associated with a high performance operating condition of said UV source (14) and the step of switching on the UV source (14) when the inner temperature of the chilling chamber (3) falls in said prefixed first temperature range.

11. Method according to claim 10, wherein said step of sanitizing comprises the step of controlling said defrost system (12) to heat the first heat exchanger (8) so as to have simultaneously the temperature of said first heat exchanger (8) in a prefixed second temperature range associated with a thermal sanitization condition of said heat exchanger (8), and the temperature of the chilling chamber (3) in said prefixed first temperature range and the step of switching on said UV source (14) when the temperature of the first heat exchanger (8) falls in said prefixed second temperature range and the inner temperature of the chilling chamber (3) falls in said prefixed first temperature range.

12. Method according to any of the Claims from 10 to 11, comprising the steps of:
• measuring the temperature inside of said chilling chamber (3);
• switching-on said UV source (14) to generate ultraviolet radiations when said measured temperature falls in said prefixed first temperature range.

13. Method according to any of the Claims from 10 to 12, wherein said prefixed first temperature range is between about 30°C and 50° C and/or said prefixed second temperature range is between about 60°C and 80° C.

## Patentansprüche

1. Schockkühlervorrichtung (1), umfassend eine Kühlkammer (3), die zum Aufnehmen von Lebensmitteln strukturiert ist, die gekühlt/gefroren werden sollen, einen Kühlkreis (6), der mindestens einen ersten Wärmetauscher (8) umfasst, der ausgelegt ist, die Kühlkammer (3) zu kühlen/zu frieren;
**dadurch gekennzeichnet, dass** sie ein Sterilisierungssystem (13) umfasst, welches konfiguriert ist, die Kühlkammer (3) durch Zerstören und/oder Deaktivieren von Mikroorganismen zu sterilisieren, welche die Kühlkammer (3) kontaminieren, das Sterilisierungssystem (13) Folgendes umfassend:
- ein Enteisungssystem (12), das ausgelegt ist, den ersten Wärmetauscher (8) zu enteisen;
- Mindestens eine UV-Quelle (14), die ausgelegt ist, die Innenflächen der Kühlkammer (3) mit ultravioletten Strahlungen zu bestrahlen; und
- ein Steuerungssystem (15), das konfiguriert ist, das Enteisungssystem (12) zu steuern, um den ersten Wärmetauscher (8) zu heizen, um so zu bewirken, dass die Innentemperatur der Kühlkammer (3) ansteigt, bis sie innerhalb eines vorbestimmten ersten Temperaturbereichs liegt, der einem Hochleistungsbetriebszustand der UV-Quelle (14) zugeordnet ist, und weiterhin konfiguriert ist, die UV-Quelle (14) einzuschalten, wenn die Innentemperatur der Kühlkammer (3) in den vorbestimmten ersten Temperaturbereich fällt.

2. Schockkühlervorrichtung nach Anspruch 1, wobei das Steuerungssystem (15) konfiguriert ist, das Enteisungssystem (12) zu steuern, um den ersten Wärmetauscher (8) zu heizen, um so gleichzeitig die Temperatur des ersten Wärmetauschers (8) in einen vorbestimmten zweiten Temperaturbereich, der einem thermischen Sterilisierungszustand des Wärmetauschers (8) zugeordnet ist, und die Temperatur der Kühlkammer (3) in den vorbestimmten ersten Temperaturbereich zu bringen oder dort zu halten, und weiterhin konfiguriert ist, um die UV-Quelle (14) einzuschalten, wenn die Temperatur des ersten Wärmetauschers (8) in den vorbestimmten zweiten Temperaturbereich fällt und die Innentemperatur der Kühlkammer (3) in den vorbestimmten ersten Temperaturbereich fällt.

3. Schockkühlervorrichtung nach Anspruch 1 oder 2, wobei das Steuerungssystem (15) mindestens einen Temperatursensor (16, 17) umfasst, der konfiguriert ist, die Temperatur innerhalb der Kühlkammer (3) zu messen; wobei das Steuerungssystem (15) weiterhin konfiguriert ist, die UV-Quelle (14) einzuschalten, um ultraviolette Strahlungen zu erzeugen, wenn die gemessene Temperatur in den vorbestimmten ersten Temperaturbereich fällt.

4. Schockkühlervorrichtung nach einem der vorhergehenden Ansprüche, wobei der vorbestimmte erste Temperaturbereich zwischen ungefähr 30 °C und 50 °C liegt.

5. Schockkühlervorrichtung nach Anspruch 2, wobei der vorbestimmte zweite Temperaturbereich zwischen ungefähr 50 °C und 80 °C liegt.

6. Schockkühlervorrichtung nach Anspruch 5, wobei der vorbestimmte zweite Temperaturbereich zwischen ungefähr 60 °C und 80 °C liegt.

7. Schockkühlervorrichtung nach einem der vorhergehenden Ansprüche, wobei der Kühlkreis (6) eine Wärmepumpeneinheit mit umkehrbarem Prozess (7) umfasst, die den ersten Wärmetauscher (8) umfasst, und entworfen ist, so zu arbeiten, dass sie einen unmittelbaren Wärmezyklus, um den ersten Wärmetauscher (8) zu kühlen, um so die Kühlkammer (3) schnell zu kühlen/zu frieren, oder alternativ einen umgekehrten Wärmezyklus durchführt, um den ersten Wärmetauscher (8) zu heizen, um so den ersten Wärmetauscher (8) zu enteisen.

8. Schockkühlervorrichtung nach einem der Ansprüche 1 bis 6, wobei der Kühlkreis (6) mit Folgendem wersehen ist
einer Wärmepumpeneinheit (7), die ein Kältemittelverdichtungsmittel (10) umfasst; einem Umgehungskanal (19), der zwischen dem Kältemittelauslass des Kältemittelverdichtungsmittels (10) und dem Kältemitteleinlass des ersten Wärmetauschers (8) angeordnet ist; und einem Ventilmittel (20), das entlang des Umgehungskanals (19) angeordnet ist, um auf der Grundlage eines Befehls den Kältemittelauslass des Kältemittelverdichtungsmittels (10) mit dem Kältemitteleinlass des ersten Wärmetauschers (8) zu verbinden;
wobei das Steuerungssystem (15) konfiguriert ist, das Ventilmittel (20) zu steuern, um den ersten Wärmetauscher (8) zu enteisen.

9. Schockkühlervorrichtung nach einem der Ansprüche 1 bis 8, wobei das Enteisungssystem (12) ein elektrisches Heizmittel (21) umfasst, das dem ersten Wärmetauscher (8) zugeordnet ist; wobei das Steuerungssystem konfiguriert ist, das elektrische Heizmittel (21) zu steuern, um den ersten Wärmetauscher (8) zu enteisen.

10. Verfahren zum Sterilisieren einer Schockkühlervorrichtung (1), umfassend eine Kühlkammer (3), die zum Aufnehmen von Lebensmitteln strukturiert ist, die gekühlt/gefroren werden sollen, einen Kühlkreis (6), der mindestens einen ersten Wärmetauscher (8) umfasst, der ausgelegt ist, die Kühlkammer (3) schnell zu kühlen/zu frieren; **dadurch gekennzeichnet, dass** es ein Sterilisierungssystem (13) gibt, das ein Enteisungssystem (12) umfasst, das ausgelegt ist, den mindestens ersten Wärmetauscher (8) zu enteisen; und
es mindestens eine UV-Quelle (14) gibt, die ausgelegt ist, die Innenflächen der Kühlkammer (3) mit ultravioletten Strahlungen zu bestrahlen; das Verfahren umfassend den Schritt des Sterilisierens der Kühlkammer (3) durch Zerstören und/oder Deaktivieren von Mikroorganismen, welche die Kühlkammer (3) kontaminieren, durch das Sterilisierungssystem (13), wobei der Schritt des Sterilisierens den Schritt des Steuerns des Enteisungssystems (12) umfasst, um den ersten Wärmetauscher (8) zu heizen, um so zu bewirken, dass die Innentemperatur der Kühlkammer (3) ansteigt, bis sie innerhalb eines vorbestimmten ersten Temperaturbereichs liegt, der einem Hochleistungsbetriebszustand der UV-Quelle (14) zugeordnet ist, und den Schritt des Einschaltens der UV-Quelle (14), wenn die Innentemperatur der Kühlkammer (3) in den vorbestimmten ersten Temperaturbereich fällt.

11. Verfahren nach den Ansprüchen 10, wobei der Schritt des Sterilisierens den Schritt des Steuerns des Enteisungssystems (12), um den ersten Wärmetauscher (8) zu heizen, um so gleichzeitig die Temperatur des ersten Wärmetauschers (8) in einen vorbestimmten zweiten Temperaturbereich, der einem thermischen Sterilisierungszustand des Wärmetauschers (8) zugeordnet ist, und die Temperatur der Kühlkammer (3) in den vorbestimmten ersten Temperaturbereich zu bringen, und den Schritt des Einschaltens der UV-Quelle (14) umfasst, wenn die Temperatur des ersten Wärmetauschers (8) in den vorbestimmten zweiten Temperaturbereich fällt und die Innentemperatur der Kühlkammer (3) in den vorbestimmten ersten Temperaturbereich fällt.

12. Verfahren nach einem der Ansprüche 10 bis 11, die folgenden Schritte umfassend:
• Messen der Temperatur innerhalb der Kühlkammer (3);
• Einschalten der UV-Quelle (14), um ultraviolette Strahlungen zu erzeugen, wenn die gemessene Temperatur in den vorbestimmten ersten Temperaturbereich fällt.

13. Verfahren nach einem der Ansprüche 10 bis 12, wobei der vorbestimmte erste Temperaturbereich zwischen ungefähr 30 °C und 50 °C liegt und/oder der vorbestimmte zweite Temperaturbereich zwischen ungefähr 60 °C und 80 °C liegt.

## Revendications

1. Appareil de réfrigération rapide (1) comprenant une chambre de réfrigération (3) structurée pour accueillir des aliments à refroidir/congeler, un circuit de réfrigération (6) comprenant au moins un premier échangeur de chaleur (8) conçu pour refroidir/congeler la chambre de réfrigération (3) ;
**caractérisé en ce qu'**il comprend un système de désinfection (13) qui est configuré pour désinfecter ladite chambre de réfrigération (3) en détruisant et/ou en inactivant des microorganismes contaminant ladite chambre de réfrigération (3), ledit système de désinfection (13) comprenant :
- un système de décongélation (12) conçu pour décongeler ledit premier échangeur de chaleur (8) ;
- au moins une source UV (14) conçue pour irradier avec des rayons ultraviolets les surfaces internes de ladite chambre de réfrigération (3) ; et
- un système de contrôle (15) configuré pour contrôler ledit système de décongélation (12) pour chauffer le premier échangeur de chaleur (8) de manière à faire grimper la température interne de la chambre de réfrigération (3) jusqu'à ce qu'elle soit comprise dans une première gamme de températures préfixée associée à une condition de fonctionnement à hautes performances de ladite source UV (14) et étant en outre configuré pour allumer ladite source UV (14) quand la température interne de la chambre de réfrigération (3) se retrouve dans ladite première gamme de températures préfixée.

2. Appareil de réfrigération rapide selon la revendication 1, dans lequel ledit système de contrôle (15) est configuré pour contrôler le système de décongélation (12) pour chauffer le premier échangeur de chaleur (8) de manière à avoir ou maintenir simultanément la température dudit premier échangeur de chaleur (8) dans une deuxième gamme de températures préfixée associée à une condition de désinfection thermique dudit échangeur de chaleur (8) et la température de la chambre de réfrigération (3) dans ladite première gamme de températures préfixée, et est également configuré pour allumer ladite source UV (14) quand la température du premier échangeur de chaleur (8) se retrouve dans ladite deuxième gamme de températures préfixée et la température interne de la chambre de réfrigération (3) se retrouve dans ladite première gamme de températures préfixée.

3. Appareil de réfrigération rapide selon la revendication 1 ou 2, dans lequel le système de contrôle (15) comprend au moins un capteur de température (16, 17) configuré pour mesurer la température à l'intérieur de ladite chambre de réfrigération (3) ; le système de contrôle (15) étant également configuré pour allumer ladite source UV (14) pour générer des rayons ultraviolets quand ladite température mesurée se retrouve dans ladite première gamme de températures préfixée.

4. Appareil de réfrigération rapide selon l'une quelconque des revendications précédentes, dans lequel ladite première gamme de températures préfixée se situe entre environ 30 °C et 50 °C.

5. Appareil de réfrigération rapide selon la revendication 2, dans lequel ladite deuxième gamme de températures préfixée se situe entre environ 50 °C et 80 °C.

6. Appareil de réfrigération rapide selon la revendication 5, dans lequel ladite deuxième gamme de températures préfixée se situe entre environ 60 °C et 80 °C.

7. Appareil de réfrigération rapide selon l'une quelconque des revendications précédentes, dans lequel ledit circuit de réfrigération (6) comprend un ensemble pompe à chaleur à cycle réversible (7), qui comprend ledit premier échangeur de chaleur (8) et est conçu pour fonctionner pour effectuer un cycle thermique direct pour refroidir ledit premier échangeur de chaleur (8) de manière à refroidir/congeler rapidement la chambre de réfrigération (3) ou, alternativement, un cycle thermique inversé, pour chauffer ledit premier échangeur de chaleur (8), de manière à décongeler ledit premier échangeur de chaleur (8).

8. Appareil de réfrigération rapide selon l'une quelconque des revendications 1 à 6, dans lequel ledit circuit de réfrigération (6) est pourvu d'un ensemble pompe à chaleur (7) comprenant un moyen de compression de réfrigérant (10) ; un canal de dérivation (19) qui est intercalé entre la sortie de réfrigérant du moyen de compression de réfrigérant (10) et l'entrée de réfrigérant dudit premier échangeur de chaleur (8) ; et un moyen formant vanne (20) disposé le long dudit canal de dérivation (19) pour raccorder, sur la base d'une commande, la sortie de réfrigérant du moyen de compression de réfrigérant (10) à l'entrée de réfrigérant du premier échangeur de chaleur (8) ; ledit système de contrôle (15) étant configuré pour contrôler ledit moyen formant vanne (20) pour décongeler ledit premier échangeur de chaleur (8).

9. Appareil de réfrigération rapide selon l'une quelconque des revendications 1 à 8, dans lequel ledit système de décongélation (12) comprend un moyen de chauffage électrique (21) associé audit premier échangeur de chaleur (8) ; ledit système de contrôle étant configuré pour contrôler ledit moyen de chauffage électrique (21) pour décongeler ledit premier échangeur de chaleur (8).

10. Procédé de désinfection d'un appareil de réfrigération rapide (1) comprenant une chambre de réfrigération (3) structurée pour accueillir des aliments à refroidir/congeler, un circuit de réfrigération (6) comprenant au moins un premier échangeur de chaleur (8) conçu pour refroidir/congeler rapidement la chambre de réfrigération (3) ; **caractérisé en ce qu'**il y a un système de désinfection (13) comprenant un système de décongélation (12) conçu pour décongeler ledit au moins un premier échangeur de chaleur (8) ; et au moins une source UV (14) conçue pour irradier avec des rayons ultraviolets les surfaces internes de ladite chambre de réfrigération (3) ; le procédé comprenant l'étape de désinfection de ladite chambre de réfrigération (3) par destruction et/ou inactivation de microorganismes contaminant ladite chambre de réfrigération (3) par le système de désinfection (13),
ladite étape de désinfection comprenant l'étape de contrôle dudit système de décongélation (12) pour chauffer le premier échangeur de chaleur (8) de manière à faire grimper la température interne de la chambre de réfrigération (3) jusqu'à ce qu'elle soit comprise dans une première gamme de températures préfixée associée à une condition de fonctionnement à hautes performances de ladite source UV (14) et l'étape d'allumage de la source UV (14) quand la température interne de la chambre de réfrigération (3) se retrouve dans ladite première gamme de températures préfixée.

11. Procédé selon les revendications 10, dans lequel ladite étape de désinfection comprend l'étape de contrôle dudit système de décongélation (12) pour chauffer le premier échangeur de chaleur (8) de manière à avoir simultanément la température dudit premier échangeur de chaleur (8) dans une deuxième gamme de températures préfixée associée à une condition de désinfection thermique dudit échangeur de chaleur (8) et la température de la chambre de réfrigération (3) dans ladite première gamme de températures préfixée, et l'étape d'allumage de ladite source UV (14) quand la température du premier échangeur de chaleur (8) se retrouve dans ladite deuxième gamme de températures préfixée et la température interne de la chambre de réfrigération (3) se retrouve dans ladite première gamme de températures préfixée.

12. Procédé selon l'une quelconque des revendications 10 à 11, comprenant les étapes consistant à :
• mesurer la température à l'intérieur de ladite chambre de réfrigération (3) ;
• allumer ladite source UV (14) pour générer des rayons ultraviolets quand ladite température mesurée se retrouve dans ladite première gamme de températures préfixée.

13. Procédé selon l'une quelconque des revendications 10 à 12, dans lequel ladite première gamme de températures préfixée se situe entre environ 30 °C et 50 °C et/ou ladite deuxième gamme de températures préfixée se situe entre environ 60 °C et 80 °C.
